# DEMANDE DE BREVET EUROPEEN

(11) **EP 0 926 153 A1**
(43) Date de publication de la demande: **30.06.1999**
(21) Numéro de dépôt: 98402918.1
(22) Date de dépôt: 24.11.1998
(51) Int. Cl.: C07H 3/02, C07H 7/033, C07H 7/00

(54) **Décarboxylation au cuivre**

(30) Priorité: 26.11.1997 FR 9714866
(71) Demandeur: Roquette Frères, 62136 Lestrem (FR)
(72) Inventeur: Tamion, Rodolphe, 62157 Allouagne (FR)
(74) Mandataire: Boulinguiez, Didier

(57) **Abrégé**

La présente invention a pour objet un procédé de fabrication d'un aldose ou d'un dérivé d'aldose comportant sur la chaîne hydrocarbonée n atomes de carbone, caractérisé par le fait que l'on met en contact une solution aqueuse d'un dérivé acide d'ose à n+1 atomes de carbone, comportant au moins un motif α-hydroxyacide, ou un de ses sels, avec du peroxyde d'hydrogène en présence d'un sel de cuivre, que l'on sépare ensuite le cuivre qui précipite du milieu, puis que l'on régénère un sel de cuivre à l'aide de l'acide correspondant et que l'on recycle ensuite le sel de cuivre ainsi régénéré en tête du procédé.

## Description

La présente invention a pour objet un procédé de fabrication d'un aldose ou d'un dérivé d'aldose.

Plus précisément, elle a pour objet un procédé de fabrication d'un aldose ou d'un dérivé d'aldose comportant sur la chaîne hydrocarbonée n atomes de carbone, à partir d'un dérivé acide d'ose à n+1 atomes de carbone présentant au moins un motif α- hydroxyacide, ou d'un de ses sels, ce procédé consistant à mettre en contact ledit dérivé acide d'ose avec du peroxyde d'hydrogène en présence d'un sel de cuivre, en phase aqueuse, à séparer le cuivre qui précipite du milieu, à régénérer un sel de cuivre à l'aide de l'acide correspondant et à recycler le sel de cuivre ainsi régénéré en tête du procédé.

Au sens de la présente invention, on convient d'entendre par :
- "aldose", un ose comportant une fonction aldéhyde ;
- "dérivé d'aldose", plus particulièrement un acide uronique (acide monocarboxylique dérivé d'un aldose par remplacement du groupement CH₂OH par un groupement COOH);
- "dérivé acide d'ose comportant au moins un motif α-hydroxyacide", un acide mono- ou dicarboxylique dérivé d'un aldose et comportant une fonction CHOH en α de la ou des fonction(s) acide(s).

Le procédé de la présente invention permet d'obtenir, avec un excellent rendement, un aldose à partir d'un acide aldonique ou un acide uronique à partir d'un acide aldarique.

Les aldoses obtenus par la mise en oeuvre du procédé conforme à l'invention sont d'un grand intérêt en tant que tels, mais seraient surtout des intermédiaires de synthèse fort importants s'il advenait que l'on puisse les produire en grande quantité et à faible coût. En effet, une étape complémentaire simple d'hydrogénation de ces aldoses permet d'obtenir facilement les alditols correspondants, qui sont tous des polyols pouvant être employés dans de multiples applications et notamment en tant que substituts non cariogènes et hypocaloriques du saccharose.

Les acides uroniques obtenus par la mise en oeuvre du procédé conforme à l'invention, en tant qu'acides polyhydroxycarboxyliques, possèdent des propriétés séquestrantes pouvant être exploitées dans le domaine des ciments, mortiers ou ciments où de tels acides ont été proposés comme retardateurs de prise, ou encore dans le domaine de la détergence, où ces acides ont été proposés pour le nettoyage d'articles en verre ou en métal ou à titre d'additifs pour détergents.

C'est en étudiant la méthode exposée par RUFF, il y a près d'un siècle (Ber. 32, 3674 (1889) ; 33, 1799 (1900)) que la Demanderesse a mis au point ce nouveau procédé de fabrication d'un aldose ou d'un dérivé d'aldose, par voie chimique, à partir d'un dérivé acide d'ose ou de ses sels.

La méthode de RUFF permet de passer, de façon générale, d'un acide aldonique comportant n carbones à un aldose comportant (n-1) carbones grâce à l'action combinée d'ions ferriques et d'eau oxygénée. Cependant, les rendements en aldose sont très médiocres.

La conversion de l'acide gluconique en D-arabinose peut ainsi être réalisée selon cette méthode.

Quelques améliorations ont, par la suite, été apportées par R.C. Hockett et C.S. Hudson (J. Amer. Chem. Soc. 56, 1632-1633, (1934) et ibid. 72,4546, (1950)) et par les inventeurs du brevet US-A 3.755.294. Des rendements de 60 % d'arabinose en partant d'acide gluconique y sont décrits. Un progrès a encore été accompli par V. Bilik (CZ - 232647, (1983)) en utilisant les ions cuivriques (Cu (II)) comme catalyseurs. Des rendements de l'ordre de 70 % sont atteints après une purification laborieuse. Sont notamment nécessaires l'addition de résines échangeuses d'ions et une chromatographie sur colonne.

Des résultats identiques ont été obtenus récemment avec un mélange d'ions ferriques et ferreux comme catalyseurs (CZ - 279002, (1994)).

Enfin, dans des conditions particulières, le document EP-A 0.716.067 rapporte des rendements de 78 % en certains aldoses, mais la technique nécessite également une chromatographie échangeuses d'ions afin d'éliminer les impuretés.

Durant une investigation apprcfondie de la réaction de Ruff, la Demanderesse a découvert que si la réaction est catalysée par des sels de cuivre, dans des conditions de pH optimisées, on peut, de façon inattendue, faire précipiter le cuivre sous forme d'oxyde à la fin de l'addition du peroxyde d'hydrogène. Conformément à l'invention, les valeurs de pH doivent être comprises entre 5 et 9 , et plus particulièrement entre 6 et 8.

La Demanderesse a tiré profit de cette observation de la précipitation du cuivre et, en poursuivant ses investigations, a découvert qu'après filtration du milieu réactionnel, celui-ci ne contient quasiment plus de cuivre (de l'ordre de 0,3 ppm) et que sous l'action d'un acide, le précipité obtenu peut régénérer un sel de cuivre pouvant être récupéré et recyclé dans une excellente proportion en tête du procédé. Le taux de récupération du catalyseur est de l'ordre de 95 %.

Le cuivre étant utilisé en proportion non négligeable, de l'ordre de 2 % en poids, le procédé conforme à l'invention permet de réaliser ainsi des économies substantielles.

En outre, il évite un inconvénient majeur des procédés décrits dans l'art antérieur, résidant dans les difficultés de purification, en simplifiant de manière déterminante les étapes de purification.

Ainsi, selon l'invention, le procédé de fabrication d'un aldose ou d'un dérivé d'aldose comportant sur la chaîne hydrocarbonée n atomes de carbone, est caractérisé par le fait que dans une première étape, l'on met en contact une solution aqueuse d'un dérivé acide d'ose à n+1 atomes de carbone comportant au moins un motif α-hydroxyacide, ou un de ses sels, avec du peroxyde d'hydrogène en présence d'un sel de cuivre, que l'on sépare ensuite l'oxyde de cuivre qui précipite du milieu, que l'on régénère un sel de cuivre à l'aide de l'acide correspondant, et que l'on recycle le sel de cuivre ainsi régénéré en tête du procédé.

Le procédé de l'invention met donc en oeuvre un dérivé acide d'ose ou un de ses sels. Dans la présente invention, comme indiqué précédemment, on entend par dérivé acide d'ose, un acide mono- ou dicarboxylique dérivé d'un aldose. Cette définition englobe en particulier :
- les acides aldoniques, qui sont des acides monocarboxyliques dérivés des aldoses par remplacement du groupement aldéhyde par un groupement carboxy, tels que les acides glucoheptonique, mannonique, idonique, galactonique, lyxonique, xylonique, arabinonique et ribonique;
- les acides aldariques, qui sont des acides dicarboxyliques dérivés des aldoses par remplacement des deux groupements terminaux (CH0 et CH₂0H) par des groupements carboxy, tels que les acides glucarique et xylarique.

Dans la présente invention, on entend par sel d'un dérivé acide d'ose, le dérivé acide sous forme libre, sous forme lactonisée ou sous forme de mélange de ces deux formes, sous forme de sels ou sous forme d'esters. Ainsi, par exemple les sels de calcium, de sodium ou les y- et δ-lactones de ces dérivés acides d'oses conviennent parfaitement.

Les acides aldoniques sont obtenus de manière connue par oxydation de l'ose correspondant. Cette étape d'oxydation peut être conduite soit par voie chimique, soit par voie microbiologique.

La voie chimique préférée dans le cadre de l'invention consiste à oxyder l'ose à l'aide d'air ou d'oxygène en milieu alcalin et à l'aide de catalyseurs au palladium.

Un procédé particulièrement préféré est celui qui a été décrit dans le document US-A 4.845.208, dont la Demanderesse est titulaire, qui consiste à utiliser comme catalyseur d'oxydation du palladium fixé sur charbon actif et dopé au bismuth.

On peut également envisager d'oxyder l'ose par voie électrolytique ou à l'aide d'hypobromite. On peut encore oxyder l'ose par voie microbiologique à l'aide de *Gluconobacter* ou *d'Aspergillus.*

Les acides aldariques peuvent être obtenus, de manière connue, par oxydation des oses correspondants en présence d'air et de platine.

De préférence, le procédé selon l'invention est mis en oeuvre, en phase aqueuse, avec une teneur en matières sèches en dérivé acide d'ose, ou en un de ses sels, comprise entre 1 et 60 %, préférentiellement entre 5 et 60 % et plus particulièrement entre 5 et 40 %.

Les contraintes de matières sèches minimales sont imposées pour des raisons évidentes d'économie d'évaporation d'eau et de réduction de la taille des réacteurs.

Les contraintes de matières sèches supérieures sont essentiellement imposées par des problèmes de solubilité ou de viscosité du milieu réactionnel.

Dans la présente description, tous les pourcentages sont exprimés par rapport au dérivé acide d'ose ou à un de ses sels (exemple : 50 mol % signifie 50 moles de X pour 100 moles de dérivé acide d'ose ou d'un de ses sels, et 50 % signifie 50 grammes de X pour 100 grammes de dérivé acide d'ose ou d'un de ses sels).

Dans le procédé conforme à l'invention, le catalyseur est constitué par des ions cuivre. L'acétate, l'acétylacétonate, les halogénures, le nitrate, le sulfate de cuivre, par exemple, conviennent parfaitement.

Une quantité de catalyseur comprise entre 0,01 et 50 %, préférentiellement entre 0,1 et 20 % et plus particulièrement entre 1 et 10 % par rapport au dérivé acide d'ose mis en oeuvre ou à un de ses sels, donne de bons résultats dans le procédé conforme à l'invention, tant en ce qui concerne le rendement que la pureté de l'aldose ou du dérivé d'aldose.

Au mélange d'acide d'ose, ou d'un de ses sels, de catalyseur et d'eau, on ajoute lentement, sous agitation, le peroxyde d'hydrogène, de préférence sous forme d'eau oxygénée d'une richesse de 35 % à 70 %, à raison de 1 à 500 mol %, préférentiellement de 50 à 400 mol % et plus particulièrement de 100 à 300 mol % par rapport au dérivé acide d'ose mis en oeuvre ou à l'un de ses sels.

Le procédé de l'invention est mis en oeuvre, grâce à de la soude, à un pH compris entre 2 et 12, de préférence entre 5 et 9 et plus préférentiellement encore entre 6 et 8.

De préférence, le procédé de l'invention est mis en oeuvre à une température comprise entre 0 et 100 °C et préférentiellement entre 10 et 50 °C.

L'addition de l'eau oxygénée est effectuée à une vitesse d'introduction telle que la température du milieu réactionnel ne s'élève pas de préférence au-delà de 50°C et plus particulièrement au-delà de 40°C. Ainsi, la vitesse d'introduction de l'eau oxygénée se situe généralement entre 30 minutes et 2 heures.

Des températures inférieures amènent des temps de réaction trop longs, et des températures supérieures, outre qu'elles nécessiteraient l'emploi de réacteurs résistants à la pression, conduiraient à une dégradation des produits de la réaction.

Les températures de 15 à 40°C sont donc particulièrement préférées dans le procédé de l'invention.

L'invention sera mieux comprise au moyen de l'exemple qui suit et qui a pour seul but de mieux illustrer l'invention sans vouloir la réduire au mode de réalisation expressément décrit et au sel de dérivé acide d'ose mis en oeuvre.

Dans l'exemple qui suit, tous les résultats sont exprimés en pourcentage molaire.

### Exemple :

L'arabinonate de sodium (188 grammes, une mole), le sulfate de cuivre pintahydrate (3,8 grammes, 15 mmoles) et de l'eau (1700 ml) sont introduits dans un réacteur double enveloppe. Le mélange est porté à une température de 25°C et à un pH de 7 par addition de soude 2M. L'eau oxygénée à 35 % (170 ml, 2 moles) est introduite en 60 minutes en maintenant la température entre 20 et 25°C et le pH à 7 à l'aide de soude 2M. A la fin de l'addition, la solution est agitée une heure supplémentaire. Pendant ce temps, un précipité rouge se forme. Le solide est filtré et transformé en sulfate de cuivre par addition d'acide sulfurique dilué et peut ainsi être réutilisé pour une nouvelle réaction. Le taux de conversion est de 100 % et le rendement molaire en D-érythrose s'établit à 80 %.

## Revendications

1. Procédé de fabrication d'un aldose ou d'un dérivé d'aldose comportant sur la chaîne hydrocarbonée n atomes de carbone, caractérisé par le fait que l'on met en contact une solution aqueuse d'un dérivé acide d'ose à n+1 atomes de carbone, comportant au moins un motif α-hydroxyacide, ou un de ses sels, avec du peroxyde d'hydrogène en présence d'un sel de cuivre, que l'on sépare ensuite le cuivre qui précipite du milieu, puis que l'on régénère un sel de cuivre à l'aide de l'acide correspondant et que l'on recycle ensuite le sel de cuivre ainsi régénéré en tête du procédé.

2. Procédé de fabrication selon la revendication 1, caractérisé par le fait que la solution aqueuse a une matière sèche en dérivé acide d'ose à n+1 atomes de carbone comportant au moins un motif α-hydroxyacide, ou en un de ses sels, comprise entre 1 et 60%.

3. Procédé de fabrication selon la revendication 1 ou 2, caractérisé par le fait que le sel de cuivre est présent en une quantité comprise entre 0,01 et 50 % exprimée par rapport au dérivé acide d'ose à n+1 atomes de carbone comportant au moins un motif α-hydroxyacide ou à un de ses sels.

4. Procédé de fabrication selon l'une quelconque des revendications 1 à 3, caractérisé par le fait que l'on utilise du péroxyde d'hydrogène, de préférence sous forme d'eau oxygénée, d'une richesse de 35% à 70%, en une quantité comprise entre 1 et 500 mol % exprimée par rapport au dérivé acide d'ose à n+1 atomes de carbone comportant au moins un motif α-hydroxyacide ou à un de ses sels.

5. Procédé de fabrication selon l'une quelconque des revendications 1 à 4, caractérisé par le fait que l'on travaille à une température comprise entre 0 en 100°C, de préférence entre 10 et 50°C, et plus particulièrement entre 15 et 40°C.

6. Procédé de fabrication selon l'une quelconque des revendications 1 à 5, caractérisé par le fait que l'on travaille à un pH compris entre 5 et 9, et plus particulièrement entre 6 et 8.

7. Procédé de fabrication d'un aldose selon l'une quelconque des revendications 1 à 6, caractérisé par le fait que le dérivé acide d'ose à n+1 atomes de carbone comportant au moins un motif α-hydroxyacide est un acide aldonique ou un de ses sels.

8. Procédé de fabrication d'un aldose selon la revendication 7, caractérisé par le fait que le dérivé acide d'ose à n+1 atomes de carbone comportant au moins un motif α-hydroxyacide est l'acide arabinonique.

9. Procédé de fabrication d'un aldose selon la revendication 7, caractérisé par le fait que le sel de dérivé acide d'ose à n+1 atomes de carbone comportant au moins un motif α-hydroxy acide est l'arabinonate de sodium.

10. Procédé de fabrication d'un aldose selon la revendication 7, caractérisé par le fait que le sel d'acide d'ose à n+1 atomes de carbone comportant au moins un motif α-hydroxyacide est l'arabinonate de calcium.

11. Procédé de fabrication d'un aldose selon l'une quelconque des revendications 1 à 6, caractérisé par le fait que le dérivé acide d'ose à n+1 atomes de carbone comportant au moins un motif α-hydroxyacide est un acide aldarique.

12. Procédé de fabrication selon l'une quelconque des revendications 1 à 11, caractérisé par le fait que l'on recycle le cuivre sous l'action de l'acide sulfurique pour régénérer le sulfate de cuivre.
